(19) Europäisches Patentamt
European Patent Office
Office européen des brevets

(11) Veröffentlichungsnummer: **0 005 810**
**B1**

(12) **EUROPÄISCHE PATENTSCHRIFT**

(51) Int. Cl.³: **C 07 B 9/00**, C 07 C 41/00, C 07 C 43/12, C 07 C 45/00, C 07 C 49/16, C 07 C 68/02, C 07 C 69/96, C 07 C 51/58, C 07 C 53/48, C 07 C 59/125, C 07 C 76/02

(21) Anmeldenummer: **79101621.5**

(22) Anmeldetag: **28.05.79**

# (54) Verfahren zur Herstellung organischer Fluorverbindungen

(30) Priorität: **01.06.78 DE 2823969**

(43) Veröffentlichungstag der Anmeldung:
**12.12.79 Patentblatt 79/25**

(45) Bekanntmachung des Hinweises auf die Patenterteilung:
**12.11.80 Patenblatt 80/23**

(84) Benannte Vertragsstaaten:
**DE FR GB IT**

(56) Entgegenhaltungen:
**FR - A - 2 080 448**

(73) Patentinhaber: **HOECHST AKTIENGESELLSCHAFT**
**Zentrale Patentabteilung Postfach 80 03 20**
**D - 6230 Frankfurt/Main 80 (DE)**

(72) Erfinder: **Muffler, Herbert, Dr.**
**Tucholskystrasse 14**
**D - 6000 Frankfurt/Main 70 (DE)**
**Franz, Raimund, Dr.**
**Johann Strauss Strasse 36**
**D - 6233 Kelkheim (Taunus) (DE)**

**EP 0 005 810 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung organischer Fluorverbindungen

Organische Fluorverbindungen besitzen Bedeutung beispielsweise als potentielle Inhalationsnarkotika sowie als Zwischenprodukte für die Synthese wertvoller Endstoffe wie etwa von Reaktivfarbstoffen, von Pflanzenschutzmitteln etc.

Zu ihrer Herstellung ist eine Reihe von Methoden bekannt; eine der wichtigsten Herstellungsmethoden ist der Austausch anderer Halogenatome in den entsprechenden Halogenverbindungen gegen Fluor. Eine Übersicht über die derartigen bekannten Verfahren findet sich beispielsweise in Houben-Weyl, Methoden der Organisch ·n Chemie Band V/3, G. Thieme Verlag Stuttgart 1962, sowie in M. Hudlicky, Chemistry of Organic Fluorine Compounds, 2nd Ed. 1976, Ellis Harwood Ltd.

Je nach Verbindungstyp und Reaktivität der auszutauschenden Chlor-, Brom- oder Jodatome verwendet man als Fluorierungsmittel z.B. Fluorwasserstoff gasförmig — etwa in Reaktionsöfen über festen Katalysatoren — oder flüssig — etwa in Autoklaven in Gegenwart von Antimonsalzen. Als Beispiel fü die verwendung von Fluorwasserstoff als Fluorierungsmittel sei etwa die Herstellung des Cyanurfluorids aus Cyanurchlorid mit Fluorwasserstoff bei zunächst —78°C und anschließender Fraktionierung genannt (G. A. Olah, M. Nojima, I. Kerekes, Synthesis 1973, 487). Schon die Durchführung dieser Reaktion in relativ kleinem Maßstab erfordert jedoch einen erheblichen aparativen und sicherheitstechnischen Aufwand, da bekanntermaßen das Arbeiten mit freiem Fluorwasserstoff immer erhebliche Sicherheitsvorkehrungen erfordert.

Auch Alkalifluoride und Ammoniumfluorid sind als Fluorierungsmittel verwendet worden. Die Abstufung von deren Reaktivität läßt sich nach Hudlicky wie folgt wiedergeben:

$$CsF > RbF > KF > NaF > NH_4F > LiF$$

Demnach besitzen Cäsium- und Rubidiumfluorid innerhalb dieser Reihe die größte Fluorierungsreaktivität; diese Substanzen sind jedoch in dieser Reihe auch die teuersten. Das billigere Natrium- und Ammoniumfluorid sowie das — ebenfalls nicht sehr teure — Lithiumfluorid sind zu wenig reaktiv, was vor allem fü die beiden letztgenannten Fluoride zutrifft. Daher kommt — wenn man in der Reihe der Alkalifluoride einschließlich des Ammoniumfluorids die Reaktivität zusammen mit dem Preis berücksichtigtdem Kaliumfluorid die größte praktische Bedeutung zu.

Sehr häufig werden mit Kaliumfluorid als Fluorierungsmittel für entsprechende organische Halogenverbindungen jedoch nur unwirtschaftlich niedrige Ausbeuten an den gewünschten organischen Fluorverbindungen erzielt. So ist beispeilsweise Fluoraceton aus Bromaceton mit Kaliumfluorid in Glykol nur mit einer Ausbeute von 25 % d.Th. erhältlich (H. Machleidt, Liebigs Ann. Chem. *667*, 24 (1963)).

Die in der US—PS 3 769 434 zur Herstellung von Monofluoraus Monochlormethyle thern empfohlene Methode mit Kaliumfluorid in N-Methylpyrrolidon ist insbesondere auf die Umsetzung empfindlicher Ether nicht oder nur mit sehr geringen Ausbeuten anwendbar; die Methode versagt beispielsweise im Falle des Versuches der Herstellung des empfindlichen Cyclopropyl - monofluormethylethers

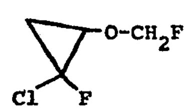

aus dem entsprechenden Monochlormethylether weitgehend. Möglicherweise ist das Versagen dieser Methode bei der Umsetzung und Herstellung empfindlicher Ether dadurch bedingt, daß hierbei relativ hohe Temperaturen angewandt werden müssen, um das in den N-Methylpyrrolidon relativ schwerlösliche anorganische Fluorid, dessen Korngröße eine erhebliche Rolle spielt, sur Reaktion zu bringen. Die relativ hohen Temperaturen führen dann häufig zur Zersetzung sowohl des Substrates als auch des Produktes und sind außerdem noch ziemlich energientensiv.

Weitere anorganische Fluorierungsmittel sind schließlich auch noch die Fluoride etwa des Silbers, des Quecksilbers und des Antimons. Deren Einsatz im großtechnischen Maßstab steht jedoch in erster Linie ihre relativ schwere Zugänglichkeit und damit ihr relativ hoher Preis entgegen.

Außer den genannten rein anorganischen Fluorierungsmitteln kennt man aber auch Fluorierungsmittel mit zumindest teilweise organischem Charakter. Solche Fluorierungsmittel sind z.B. Tetraalkylammoniumfluoride (vgl. J.—F. Normant, J. Bernadin, Compt. rend. Ser. C, *296*, 2352 (1969)). Diese Verbindungen sind jedoch, ähnlich wie etwa die vorher genannten Silber-, Quecksilber- und Antimonfluoride, relativ schwer zuganglich und deswegen ziemlich kostenungünstig.

Wegen der verschiedenen Nachteile der bekannten Fluorierungsmethoden und -mittel war es daher wünschenswert und bestand die Aufgabe, eine Fluorierungsmethode und/oder ein Fluorierungsmittel zu finden, welches diese Nachteile nicht mehr aufweist. Es sollte also inbesondere das Arbeiten mit größeren Mengen freier Flußsäure vermieden werden; außerdem sollte die neue Methode

bzw. das neue Mittel leicht zugänglich und damit preisgünstig, und auch für die Herstellung empfindlicher fluorierter Substanzen andwendbar sein.

Diese Aufgabe konnte erfindungsgemäß auf einfache Weise durch den Einsatz von Hydrofluoriden organischer Stickstoffbasen als Fluorierungsmittel gelöst werden.

Erfindungsgegenstand ist somit ein Verfahren zur Herstellung organischer Fluorverbindungen durch Umsetzung der entsprechenden Halogenverbindungen, welche an wenigstens einer Position durch von Fluor verschiedenes Halogen substituiert sind, mit Fluoriden von Stickstoffbasen; das Verfahren ist dadurch gekennzeichnet, daß als Fluoride von Stickstoffbasen die Hydrofluoride organischer Stickstoffbasen verwendet werden.

Für das Verfahren können im Prinzip alle möglichen organischen Ausgangshalogenverbindungen, welche an wenigstens einer Position durch nukleophil substituierbares Chlor, Brom ud/oder Jod substituiert sind, eingesetzt werden, doch ist es bevorzugt, nur die entsprechenden Chlor- und/oder Bromverbindungen zu verwenden.

Besonders bevorzugt ist die Verwendung der folgenden Verbindungen bzw. Verbindungsklassen:

Chlor- und/oder Brom-alkane (geradkettig oder verzweight), vorzugsweise mit 1 bis 10, insbesondere mit 2 bis 4 C-Atomen, wie z.B. Ethylbromid, i-Propylchlorid, n-Butyl-bromid und n-Octylbromid;

$\alpha$-Chlor- und/oder $\alpha$-Brom-ether, bei denen die Reste beiderseits des Ethersauerstoffatoms gleich oder ungliech sein können und vorzugsweise je 1 bis 10, insbesondere 1 bis 6 C-Atome enthalten, wie z.B. 1-Bromethyl-2-chlorethylether, 2-H-Hexafluorpropyl-chlormethylether, 2.2.2'-Trifluor-1-chlorethyl-ethylether und 1.2'-Di-chlor-2-F-cyclopropyl-n-pentylether;

$\alpha$-Chlor- und/oder $\alpha$-Bromketone, vorzugsweise (insgesamt) mit 3 bis 14, insbesondere 3 bis 8 C-Atomen, wie z.B. Monobromaceton, $\alpha$-Chlorethyl-ethylketon und $\alpha$-Bromacetophenon;

$\alpha$-Chlor- und/oder $\alpha$-Brom-carbonsäureester, vorzugsweise mit 2 bis 10, insbesondere mit 2 bis 5 C-Atomen, wie z.B. Monochloressigsäureethylester und Monobromessigsäure-i-propylester; Carbonsäurechloride und/oder -bromide, vorzugsweise mit 2 bis 12, insbesondere mit 2 bis 8 C-Atomen, wie z.B. Propionylbromid und Benzylchlorid; sowie Chlor- und/oder Brom-aromaten und/oder -heterocylen, wie z.B. 2.4-Dinitrochlorbenzol oder 2-Chlorbenzthiazol.

Durch die erfindungsgemäße Umsetzung werden in diesen Ausgangsverbindungen dann je nach den angewandten Reaktionsbedingungen und Mengenverhältnissen ein oder mehr Halogenatome pro Molekül durch Fluoratome ersetzt.

Das wesentliche Merkmal der Erfindung besteht in der Verwendung von Hydrofluoriden organischer Stickstoffbasen als Fluorierungsmittel. Die Hydrofluoride können durch die allgemeine Formel I

$$B \cdot (HF)_n \qquad (I)$$

wiedergegeben werden, worin B eine organische Stickstoffbase und n ganze oder gebrochene Zahlen von 1 bis 4 bedeuten.

Als organische Stickstoffbasen B kommen alle möglichen primären, sekundären und/oder tertiären Amine einschließlich N-Heterocyclen infrage. Wenn man als allgemine Formel für diese Amine die Formel II

$$R_1R_2R_3N \qquad (II)$$

angibt, können darin bedeuten:

$R_1$: einen Alkylrest, vorzugsweise mit 1 bis 10, insbesondere mit 1 bis 6 C-Atomen,
einen Cycloalkylrest, vorzugsweise mit 5 bis 7 C-Atomen,
einen Aralkylrest, vorzugsweise mit 6 bis 10 C-Atomen oder
einen Arylrest, vorzugsweise ebenfalls mit 6 bis 10 C-Atomen;

$R_2$ und $R_3$: Wasserstoff,
Alkyl-, Cycloalkyl-, Aralkyl und Arylreste der gleichen Art wie bei $R_1$ angegeben.

Die Reste $R_2$ und $R_3$ können gleich oder verschieden sein. Zwei der Reste $R_1$ und $R_2$ oder $R_3$ können auch zu einem cycloaliphatischen Ring, welcher gegebenenfalls noch durch andere Heteroatome wie durch Sauerstoffatome unterbrochen sein kann, geschlossen sein. Ebenfalls ist es möglich, daß die drei Reste $R_1$, $R_2$ und $R_3$ Bestandteil eines heterocyclischen Ringes sind, wodurch dann entsprechende N-Heterocyclen resultieren. Bevorzugte organische Stickstoffbasen B sind primäre, sekundäre und/oder tertiäre Amine mit insgesamt bis zu 12 C-Atomen, wobei die sekundären und/oder tertiären aliphatischen Amine besonders bevorzugt sind.

Konkrete Beispiele für die Basen B sind:

N-Butylamin, N-Decylamin, Diethylamin, Di-n-octylamin, Trimethylamin, Triethylamin, Trio-n-propylamin, Isopropyldiethylamin, Tri-n-butylamin, Cyclohexylamin, N-Methylanilin, N,N-Dimethylanilin, Pyrrolidin, Piperidin, N-Methylpiperidin, Morpholin, Pyridin, Chinolin etc.

Die Hydrofluoride I der Stickstoffbasen B sind leicht aus den Basen B und Fluorwasserstoff erhältlich; sie sind niedrigschmelzende, oder bei Raumtemperatur flüssige Substanzen mit beträchtlicher

thermischer Belastbarkeit; auch diese letztere Eigenschaft macht sie dem Fluorwasserstoff als Fluorierungsmittel überlegen, da auch reaktionsträgere Substrate auf einfache Weise drucklos fluoriert werden können. Die Trishydrofluoride sind sogar unzersetzt vakuumdestillierbar.

Die Hydrofluoride sind außerdem mischbar mit den meisten Lösungsmitteln und den zu fluorierenden Substraten und reaktionsfähiger als z.B. Kaliumfluorid. Da sie neutral bis höchstens schwach sauer reagieren, greifen sie Borsilikatglas auch in der Hitze praktisch nicht an und/sind entsprechend einfach und gefahrlos zu handhaben. Daher lassen sich die Fluorierungsreaktionen mit den Hydrofluoriden in Apparaturen aus Borsilikatglas durchführen.

Das erfindungsgemäße Verfahren wird durch die folgende allgemeine Reaktionsgleichung beschrieben:

$$\text{R-Hal} + \text{B}\cdot(\text{HF})_n \longrightarrow \text{R-F} + \text{B]1HHal} + (n-1)\,\text{HF}$$

R = organischer Rest

n = 1-4

Hal = Cl, Br, (J)

Das dabei angewandte Mengenverhältnis von Substrat zu Fluorierungsmittel müßte demnach theoretisch 1 zu 1 (Molverhältnis) sein. Vorzugsweise wird jedoch ein Überschuß an Fluorierungsmittel bis zu etwa 50 Molprozent, insbesondere von etwa 10 bis 20 Molprozent, verwendet. Es ist aber auch möglich, das Fluorierungsmittel als Reaktionsmedium, d.h. im großen Überschuß, einzusetzen. Die letztgenannte Verfahrensweise ist dann bevorzugt, wenn das Reaktionsgemisch mit dem als Nierderschlag anfallenden Hydrohalogenid rührbar erhalten werden soll. Dies kann aber auch durch Zusatz eines neutralen Lösungsoder Verdünnungsmittels wie z.B. von Dibutylether, Diphenylether, Dimethylsulfoxid, Sulfolan oder Diethylenglykol-dimethylether, erreicht werden.

Wenn bei der erfindungsgemäßen Umsetzung ein Monohydrofluorid verwendet wird (in Formel I n = 1), wird das im Austausch bei der Reaktion frei werdende, von Fluor verschiedene Halogen, in Form von Aminhydrohalogenid gebunden.

Wenn aber ein Amin-hydrofluorid I mit n größer als 1 eingesetzt wird, entstehen im Lauf der Reaktion (n-1) Mole Fluorwasserstoff. Daher wird in diesem Fall zweckmäßig zumindest eine solche Menge weiteres Amin zugegeben, die ausreicht, um den in Freiheit gesetzten Fluorwasserstoff während des Reaktionsablaufes erneut zu komplexieren. Dadurch ist es möglich, nach Zugabe von weiterem Substrat im Laufe des Ansatzes den eingesetzten Fluorwasserstoff weitestgehend auszunutzen.

Eine bevorzugte Verfahrensvariante, um den bei der Reaktion frei werdenden Fluorwasserstoff zu binden, besteht darin, in Gegenwart eines Lösungsmittels ausreichender Basizität zu arbeiten. Diese Variante ist vor allem dann bevorzugt, wenn die Möglichkeit einer Reaktion des Substrats oder des Produktes mit der freien Stickstoffbase B besteht. Als Lösungsmittel mit aureichender Basizität kommen insbesondere Säureamide zer Anwendung, von denen Lactame und N-alkylsubstituierte Lactame, insbesondere N-($C_1$-$C_4$)-alkylsubstituierte $C_5$- und/oder $C_6$-Lactame bevorzugt sind. Beispiele für solche Lactame und N-substituierte Lactame sind Pyrrolidon, Caprolactam, N-Methylpyrrolidon, N-Propylpyrrolidon, N-Methylcaprolactam etc.

Die erhaltenen Säureamid-Fluorwasserstoff-Gemische sind uber weite Konzentrationsbereiche hinweg unzersetzt vakuumdestillierbar. Sie können so von gelöstem Aminhydrohalogenid befreit, mit Lauge titriert und nach Zugabe der berechneten Menge Amin wieder verwendet werden.

Die Reihenfolge des Zusammengebens der Reaktanten ist nach Gesichtspunkten der Zweckmäßbigkeit beliebig wählbar. Es ist nicht unbedingt erforderlich, daß das Fluorierungsmittel bereits als fertiger Basen-Fluorwasserstoff-Komplex zum Einsatz kommt. Dieser kann gegebenenfalls auch während des Reaktionsablaufes in situ erzeugt werden. Bei dieser Verfahrensvariante wird in ein Gemisch aus Lösungsmittel, Fluorwasserstoff und Substrat Amin eingetropft. Werden als Lösungsmittel die vorstehend beschriebenen Säureamide verwendet, so lassen sich auch diese Reaktionen in Borsilikatglas durchführen.

Die Reaktionstemperaturen liegen je nach Reaktivität des Substrats zwischen etwa −10 und +150°C.

Die Reaktionsdauer ist ebenfalls abhängig von der Reaktivität des Substrates. Das Reaktionsende ist bei den Reaktionen, bei welchen das Produkt kontinuierlich abdestilliert werden kann, ohne weiteres erkennbar. In anderen Fällen kann man durch argentometrische Bestimmung der abgespaltenen Halogenionen Aufschluß über den Fortgang der Reaktion erhalten. Die Aufarbeitung des Reaktionsansatzes erfolgt auf bekannte Weise, also etwa durch Destillation, Extraktion etc.

Das erfindungsgemäße Verfahren zeichnet sich dadurch besonders aus, daß die Fluorierungsmittel einfach und billig herstellbar und gefahrlos zu handhaben sind, wodurch die Reaktion in Apparaturen aus Borsilikatglas durchgeführt werden können. Außerdem ermöglichen die erfindungsgemäßen Fluorierungssubstanzen vielfach kürzere Reaktionszeiten als nach den Verfahren des Standes der Technik, sowie mildere Bedingungen und vor allem höhere Ausbeuten.

Es war auch in keiner Weise naheliegend, organische Stickstoffbasen als Fluorierungsmittel in der erfindungsgemäßen Weise zu verwenden, da das einzige als Fluorierungsmittel bekannte Mono Hydrofluorid einer Stickstoffbase, nämlich das Ammoniumfluorid $NH_4F$, bekanntermaßen eine ausgesprochen geringe Fluorierungsreaktivität besitzt. Man hätte demnach unter den Hydrofluoriden von Stickstoffbasen überhaupt keine einigermaßen fluorierungsreaktiven Substanzen mehr vermuten

sollen. Um so überraschender war, es, als beim erfindungsgemäßen Beschreiten dieses nicht naheliegenden Weges gefunden wurde, daß die Hydrofluoride von organischen Stickstoffbasen dennoch ausgezeichnete Fluorierungsmittel darstellen.

Die als Fluorierungsmittel bekannten Tetraalkylammoniumfluoride sind keine Hydrofluoride und stehen insofern der Erfindung ferner.

Die folgenden Beispiele sollen das erfindungsgemäße Verfahren nähren näher erläutern:

Beispiel 1

In einem Kolben mit Rührer, Thermometer, Rückflußkühler und Trockenrohr wurden 217 g (1,0 Mol) 2H-Perfluor-n-propyl-chlor-methyle ther, $CF_3CHFCF_2OCH_2Cl$, und 15,6 g (1,3 Mol) Triethylaminhydrofluorid 8 Stunden bei 90°C Badtemperatur gerührt. Das Hydrofluorid ging beim Erwärmen in Lösung. Nach 1,5 Stunden begann Triethylaminhydrochlorid auszufallen. Nach Beendigung der Reaktion wurden aus dem Reaktionskolben die destillierbaren Anteile über eine 50 cm lange Vigreux-Kolonne vom ausgefallenen Salz abdestilliert. Kp.: 66°—68°C.

Ausbeute: 120 g $CF_3CHFCF_2OCH_2F \triangleq$ 60% d. Th.

Beispiel 2a

In einem Kolben mit Rührer, Thermometer und 80 cm langer Vigreux-Kolonne mit Rückflußkühler wurde unter Feuchtigkeitsausschluß eine Mischung aus 217 g (1,0 Mol) 2H-Perfluor-n-propyl-chlormethylether, $CF_3CHFCF_2OCH_2Cl$, und 137 g (1,3 Mol) Piperidinhydrofluorid so erwärmt, daß kontinuierlich 2H-Perfluor-n-propyl-fluormethylether, Kp.: 66°—68°C, abgenommen werden konnte. Nach 6 Stunden war die Reaktion beendet.

Ausbeute: 129 g $CF_3CHFCF_2OCH_2F \triangleq$ 65% d. Th.

Beispeil 2b (Vergleichsbeispiel mit Kaliumfluorid)

216,5 g (1,0 Mol) 2H-Perfluor-n-propyl-chlormethylether, $CF_3CHFCF_2OCH_2Cl$, werden mit 87 g (1,5 Mol) trockenem Kaliumfluorid in 400 ml N-Methyl-pyrrolidon 10 Stunden lang unter Feuchtigkeitsausschluß unter Rückfluß gekocht. Nach dem Abkühlen auf Raumtemperatur wird die Reaktionsmischung in Wasser gegossen, die organische Phase abgetrennt und mit Natriumsulfat getrocknet. Die Destillation über eine 50 cm lange Füllkörper-Kolonne mit 4 mm Raschig-Ringen liefert 48 g (24% d. Th.) Fluormethylether, $CF_3CHFCH_2OCH_2F$, vom Kp.: 68°—69°C.

Beispiel 3

In einem Kolben mit Rührer, Thermometer, Tropftrichter und 100 cm langer Vigreux-Kolonne wurden unter Feuchtigkeitsausschluß zu einer auf 120°C erwärmten Lösung von 83 g (1,0 Mol) n-Butylaminhydrofluorid in 100 ml N-Methylpyrrolidon 119 g (0.8 Mol) 1-Chlor-2,2,2-trifluorethylmethylether, $CF_3CHClOCH_3$, so zugetropft, daß kontinuierlich 1,2,2,2-Tetrafluor thylmethyläther abgenommen werden konnte. Reaktionszeit: 100 Minuten.

Ausbeute: 84 g $CF_3CHFOCH_3 \triangleq$ 80% d. Th.

Beispiel 4

In der gleichen Apparatur wie in Beispiel 3 beschrieben wurden 1600 g (10,8 Mol) 1-Chlor-2,2,2-trifluorethyl-methylether, $CF_3CHClOCH_3$, zu einer Lösung von 1500 g (14,3 Mol) Piperidinhydrofluorid in 1300 ml N-Methylpyrrolidon getropft. Die Reaktionstemperatur lag zwischen 135° und 150°C, die Reaktion dauerte 7 Stunden. 4 Stunden nach Beginn des Zutropfens begann Piperidinhydrochlorid auszufallen. Die Zutropfgeschwindigkeit wurde so geregelt, daß 1,2,2,2-Tetrafluorethyl-methylether, Kp.: 36°—38°C, kontinuierlich abgenommen werden konnte.

Ausbeute: 1140 g $CH_3CHFOCH_3 \triangleq$ 80% d. Th.

Beispiel 5

In einem Kolben mit Rührer, Thermometer, 80 cm langer Vigreuz-Kolonne mit Kolonnenkopf wurden unter Feuchtigkeitsausschluß 83 g (0,5 Mol) 1,2,2,2-Tetrafluorethyl-chlormethylether, $CF_3CHFOCH_2Cl$, mit 80 g (0,76 Mol) Piperidinhydrofluorid so erhitzt (Innentemperatur 85°—95°C), daß kontinuierlich 1,2,2,2-Tetrafluorethyl-fluormethylether, Kp.: 42°—44°C, abgenommen werden konnte.

Ausbeute: 52 g $CF_3CHFOCH_3F \triangleq$ 70% d. Th.

Beispiel 6

In einem 4000 ml Polyethylen-Gefäß wurde unter Feuchtigkeitsausschluß zu 1300 ml N-Methylpyrrolidon 300 g (15,0 Mol) Fluorwasserstoff unter Kühlen und Rühren zugetropft. Danach wurde bei 0°—10°C 1275 g (15,0 Mol) Piperidin zugetropft. Nach 1 Stunde Rühren bei Raumtemperatur wurde das in N-Methyl-pyrrolidon aufgeschlämmte Piperidinhydrofluorid in eine Glasapparatur gefüllt und wie in Beispiel 4 beschrieben mit 1634 g (11,0 Mol) 1-Chlor-2,2,2, trifluorethyl-methylether, $CF_2CHClOCH_3$, zum 1,2,2,2-Tetrafluorethyl-methylether, $CF_3CHFOCH_3$, umgestezt. Kp.: 37°—38°C, Ausbeute: 1152 g $CF_3CHFOCH_3 \triangleq$ 80% d. Th.

### Beispiel 7

In einem Kolben mit Rührer, Thermometer, Rückflußkühler und Trockenrohr wurden 92 g (0,5 Mol) 1-Chlor-2,2,2-trifluorethylchlormethylether, $CF_3CHClOCH_2Cl$, und 97 g (0.8 Mol) Triethylaminhydrofluorid 10 Stunden bei 90°C Badtemperatur gerührt. Aus der beim Anwärmen entstehenden homogenen Lösung begann nach 15 Minuten Triethylaminhydrochlorid auszufallen. Die Destillation des Reaktionsproduktes lieferte 67 g 1-Chlor-2,2,2-trifluorethyl-fluormethylether ≙ 80% d. Th., Kp.: 63°C.

### Beispiel 8

In einer Destillationsapparatur mit Rührer und 80 cm langer Vigreux-Kolonne wurden 183 g (1,0 Mol) 1-Chlor-2,2,2-trifluorethyl-chlormethylether, $CF_3CHClOCH_2Cl$, und 137 g (1,3 Mol) Piperidinhydrofluorid so erwärmt (die Innentemperatur stieg von 75° auf 110°C an), daß kontinuierlich 1-Chlor-2,2,2-trifluorethyl-fluormethylether, $CF_3CHClOCH_2F$, Kp.: 62°C—63°C, abgenommen werden konnte.

Ausbeute: 117 g $CF_3CHClOCH_2F$ ≙ 70% d. Th.

### Beispiel 9

In einer Destillationsapparatur mit Rührer und 80 cm langer Vigreux-Kolonne wurden unter Feuchtigkeitsausschluß 310 g (1,7 Mol) 1-Chlor-2,2,2-trifluorethyl-chlormethylether, $CF_3CHClOCH_2Cl$, 274 g (2,6 Mol) Piperidinhydrofluorid und 300 ml Xylol 7 Stunden lang so erwärmt (die Innentemperatur stieg von 105° auf 125°C an), daß kontinuierlich 1-Chlor-2,2,2-trifluorethyl-fluormethylether abgenommen werden konnte.

Ausbeute: 182 g $CF_3CHClOCH_2F$ ≙ 65% d. Th., Kp.: 63°—64.

### Beispiel 10

In einem 500 ml Stahlautoklaven mit Hub-Schub-Rührer wurden 130 g (0,7 Mol) 1-Chlor-2,2,2-trifluroethyl-chlormethylether, $CF_3CHClOCH_2Cl$, und 188 g (1,8 Mol) Piperidinhydrofluorid 5 Stunden bei 130°C Innentemperatur gerührt. Der Druck stieg auf maximal 5 bar. Die Destillation des Reaktionsproduktes über eine 80 cm lange Vigreux-Kolonne lieferte 48 g 1,2,2,2-Tetrafluorethylfluormethylether, $CF_3CHFOCH_2F$, kp.: 43°C, was einem Umsatz von 47% entspricht, sowie 37 g 1-Chlor-2,2,2-trifluorethyl-fluormethylether, $CF_3CHClOCH_2F$, Kp.: 63°C, was einem Umsatz von 32% entspricht.

### Beispiel 11.

In einer Destillationsapparatur mit 50 cm langer Vigreux-Kolonne wurde unter Rühren aus einer bei 90°—100°C gehaltenen Lösung von 157 g (1,3 Mol) Triethylaminhydrofluorid und 137 g (1,0 Mol) n-Butylbromid in 100 ml N-Methylpyrrolidon kontinuierlich n-Butylfluorid abdestilliert, Kp.: 31°—32°C.

Ausbeute: 37 g $n\text{-}C_4H_9F$ ≙ 49% d. Th.

### Beispiel 12

Eine Mischung aus 122,5 g (1,0 Mol) Chloressigsäureethylester und 157 g (1,3 Mol) Triethylaminhydrofluorid wurde unter Feuchtigkeitsausschluß 3 Stunden bei 80°C gerührt. Anschließend wurde über eine 80 cm lange Vigreux-Kolonne destilliert, Kp.: 79°—80°C/200 Torr.

Ausbeute: 74 g $FCH_2—COOC_2H_5$ ≙ 70% d.Th.

### Beispiele 13 (a-c)

Umsetzung von Chlorcarbonyl-Verbindungen

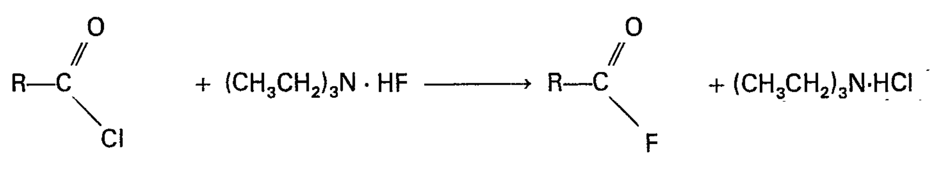


| | R | Kp. (°C) | Ausbeute (% d. Th.) |
|---|---|---|---|
| a) | $C_6H_5$ | 157 | 70 |
| b) | $CF_2Cl$ | –21 bis –19 | 70 |
| c) | $OCH_2CH_3$ | 54—56 | 75 |

In einem Kolben mit Rührer, Thermometer, Tropftrichter und Kolonne mit Kolonnenkopf wurde Triethylaminhydrofluorid in N-Methylpyrrolidon aufgeschlämmt. Zu der Aufschlämmung wurde bei 15°—20°C die Chlorcarbonyl-Verbindung zugetropft und anschließend im Vakuum alle flüchtigen Bestandteile abdestilliert. Die erhaltenen Destillate wurden redestilliert.

Beispiel 14

In einer Destillationsapparatur mit 80 cm langer Vigreux-Kolonne, Rührer, Thermometer und Tropftrichter wurde bei 135° bis 145°C zu einer Lösung von 140 g (1,1 Mol) N-Methylanilinhydrofluorid in 300 ml N-Methylpyrrolidon 119 g (0,8 Mol) 1-Chlor-2,2,2-trifluorethyl-methylether, $CF_3CHClOCH_3$, getropft. Der entstehende 1,2,2,2-Tetrafluorethyl-methylether, $CF_3CHFOCH_3$, wurde kontinuierlich abgenommen. Die Reaktionszeit betrug 4 Stunden. Es wurden 81 g Destillat vom Kp.: 38°—43°C erhalten. Die Redestillation über eine 80 cm lange Vigreux-Kolonne lieferte 61 g $CF_3CHFOCH_3$ und 15 g Ausgangsprodukt. Unter Berücksichtigung der zurückgewonnenen Ethermenge entspricht die Ausbeute 70% d. Th.

Beispiel 15

In einer Rührapparatur aus Borsilikatglas wurde ein Gemisch aus 184 g (1,14 Mol) Triethylamin-tris-hydrofluorid und 137 g (1,0 Mol) Bromaceton bei 260—270 mbar auf 110°—114°C erwärmt. Vom Beginn der Entwicklung von Fluoraceton an wurden 67 g Triethylamin (0,66 Mol) so zugetropft, daß nur wenig davon in den Gasraum über dem Reaktionsgemisch gelangte und dieses stets schwach sauer blieb. Das Fluoraceton destillierte durch eine Kolonne mit Raschigringen kontinuierlich in eine gekühlte Vorlage. Danach wurde das Reaktionsgemisch mit 200 ml trockenem Diglyme sowie weiteren 91 g (0,66 Mol) Bromaceton versetzt und abermals Fluoraceton im Vakuum abdestilliert, Während dieser Zeit wurden weitere 51 g (0.5 Mol) Triethylamin langsam zugetropft. Man erhielt so 125 g eines annähernd 87-%igen Rohproduktes, das nach Trocknen über Magnesiumsulfat und Destillieren 81 g Fluoraceton vom Kp. 78°C ergab.

Ausbeute: 64% d. Th.

Beispiel 16

Eine Mischung aus 47,5 g 2-Fluor-2-chlorcyclopropyl-chlormethylether (0,3 Mol), 24,5 g Tri-n-butylamin-tris-hydrofluorid und 37 g Tributylamin (0,2 Mol) wurde langsam unter Vakuum auf 80° bis 100°C erwärmt. Es destillierten bei einer Kopftemperatur von 40°C und einem steigenden Vakuum von 100—30 mbar 31 g eines rd. 93-%igen Produktes ab, was einer Ausbeute von 68% d. Th. entspricht. Das Produkt war ein Gemisch zweier Diastereomerer im Verhältnis von ca. 1:4 und besaß einen Siedepunkt von 35°C bei 40 mbar. Das Produkt war nach Analyse und Kernresonanzspektren identifizierbar als 2-Fluor-2-chlorcyclopropyl-fluormethylether:

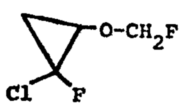

Beispiel 17

Ein Gemisch aus 2,4-Dinitrobrombenzol (1,0 Mol), 64 g Triethylamin-tris-hydrofluorid (0,4 Mol) und 81 g Triethylamin (0,8 Mol) wurde unter Rühren auf 130°C erwärmt. Nach 1 Stunde ergab die Titration einer Probe einen Gehalt an Bromionen von 87% d. Th. Nach dem Erkalten wurde das Reaktionsgemisch mehrmals mit Ether extrahiert und die Etherphase destillativ aufgearbeitet.

Ausbeute: 69 g (74% d. Th.) 2,4-Dinitrofluorbenzol vom Kp. 120°C bei 0,04 mbar; das Produkt war einheitlich nach Dünnschichtchromatogramm.

Beispiel 18

In eine Lösung von 46 g 2,4,6-Trichlorpyrimidin (0,25 Mol) in 300 g trockenem N-Methylpyrrolidon wurden bei Raumtemperatur 120 g Triethylamin-tris-hydrofluorid (0,75 Mol) getropft. Das Gemisch wurde dann innerhalb von 5 Stunden langsam bis auf 90°C erwärmt und anschließend destillativ aufgearbeitet. Man erhielt so 22 g (66% d. Th.) 2,4,6-Trilfuorpyrimidin vom Kp. 97°—102°C.

Beispiel 19

In einem Rührkolben aus Borsilikatglas legte man eine Lösung von 46 g Cyanurchlorid (0,25 Mol) in 600 ml N-Methylpyrrolidon vor und tropfte 120 g destilliertes Triethylamin-trishydrofluorid (0,75 Mol) zu. Man hielt dabei eine Temperatur von ca. 20°C durch gelegentliches Kühlen aufrecht. Es entstand alsbald ein sich allmählich verdickender Niederschlag von Triethylamin-hydrochlorid. Nach halbstündigem Nachrühren wurde der Kolben mit einem absteigenden Kühler und einer nachgeschalteten Kühlfalle im Trockeneisbad verbunden, Oelpumpenvakuum angelegt, langsam bis max. 80°C aufgeheizt und dabei die flüssigen Anteile des Reaktionsgemisches soweit wie möglich abdestilliert. Der

Vorlagekolben enthielt danach 500 g einer Lösung von 0,9 Mol HF in N-Methylpyrrolidon, die nach Zusatz von 0,3 Mol Triethylamin für den nächsten Ansatz als Fluorierungsmittel verwendbar waren. In der Kühlfalle fanden sich 30 g rohes Cyanurfluorid, aus dem bei nochmaliger Destillation 28 g (83% d. Th.) reines Produkt vom Kp. 73°C erhalten wurden.

Beispiel 20

In einem 4 Liter-Rührkolben aus Borsilikatglas mit absteigendem Kühler, 2 Liter-Vorlagekolben und nachgeschalteter Kühlfalle im Trockeneisbad wurden 2 kg einer Lösung von 300 g HF (15 Mol) in N-Methylpyrrolidon vorgelegt, 300 g Cyanurchlorid darin aufgelöst (1,63 Mol) und bei 20°—25°C unter gelegentlicher Kühlung mit Eiswasser 500 g Triethylamin (5 Mol) in ca. 2 Stunden zugetropft. Es entstand alsbald ein sich allmählich verdickender Niederschlag von Triethylamin-hydrochlorid. Man rührte eine halbe Stunde nach, legte dann Oelpumpenvakuum an, heizte langsam bis max. 80°C auf und destillierte dabei die flüssigen Anteile des Reaktionsgemisches soweit wie möglich ab. Der Vorlagekolben enthielt danach ca. 1,4 kg einer Lösung von 140 g HF (7 Mol) in N-Methylpyrrolidon; in der Kühlfalle fanden sich 205 g rohes Cyanurfluorid, aus dem bei nochmaliger Destillation 200 g (91% d. Th.) reines Produkt vom Kp. 73°C erhalten wurden.

Die destillierte Lösung von Fluorwasserstoff in N-Methylpyrrolidon wurde nach Ergänzen der verbrauchten Mengen für den nächsten Ansatz verwendet.

Der Destillationsrückstand wurde im Kolben mit verdünnter Natronlauge bis zur deutlich alkalischen Reaktion versetzt. Dabei entstand ein nahezu feststoff-freies Zweiphasengemisch, von dem das leichtere Triethylamin abgetrennt wurde. Man erhielt so 450 g, die nach Trocknung wiederverwendet wurden.

Beispiel 21

In einer Mischung aus 600 g N-Methylpyrrolidon und 90 g Fluorwasserstoff (4,5 Mol) wurden 92 g Cyanurchlorid (0.5 Mol) gelöst. Dann wurden bei 20°—30°C 278 g Tributylamin (1,5 Mol) zugetropft. Gegen Ende trübte sich die Reaktionslösung schwach durch ausfallendes Hydrochlorid. Bei 1 mbar und einer Kopftemperatur von 61°C sammelten sich anschließend 60 g rohes Cyanurfluorid in der Kühlfalle sowie 543 g N-Methylpyrrolidon mit 3,1 Mol HF in der Vorlage. Aus dem Kolbenrückstand ließ sich das Tributylamin zu 92% d. Th. zurückgewinnen. Das rohe Cyanurfluorid wurde redestilliert.

Ausbeute: 55 g (81,5% d. Th.), Kp.: 73°C.

Beispiel 22

Bei einem wie in Beispiel 21 durchgeführten Ansatz wurden als Aminkomponente 195 g N-Ethyldiisopropylamin (1,5 Mol) zugetropft. Die beschriebene destillative Aufarbeitung lieferte 538 g N-Methylpyrrolidon mit 2,4 Mol HF sowie 64 g rohes Cyanurfluorid. Nach Redestillation ergaben sich 58 g (86% d. Th.). Das Amin ließ sich zu 85% zurückgewinnen.

Beispiel 23

Bei einem wie in Beispiel 21 durchgeführten Ansatz wurden als Aminkomponente 149 g N-Methylpiperidin (1,5 Mol) zugegeben. Die beschriebene destillative Aufarbeitung lieferte 527 g N-Methylpyrrolidon mit, 2,9 Mol HF sowie 58 g Cyanurfluorid (86% d. Th.).

Beispiel 24

In einer Mischung aus 275 g N-Methylcaprolactam und 42 g Fluorwasserstoff (2,15 Mol) wurden 44 g Cyanurfluorid (0,24 Mol) gelöst. Bei 25°C wurden dann 73 g Triethylamin (0,72 Mol) eingetropft. Durch Anlegen von Oelvakuum und Erwärmen auf 70°C konnten in einer Kühlfalle 25 g Cyanurfluorid (77% d. Th.) erhalten werden.

Beispiel 25

In einer Mischung von 329 g ε-Caprolactam und 52 g Fluorwasserstoff (5,2 Mol) wurden 53 g Cyanurchlorid (0,29 Mol) gelöst. Bei 20°C wurden dann 87 g Triethylamin (0,87 Mol) eingetropft. Durch Anlegen von Oelvakuum und Erwärmen auf 70°C konnten in einer Kühlfalle 13 g Cyanurfluorid (33% d. Th.) erhalten werden.

Beispiel 26

Zu einer Lösung von 46 g Cyanurchlorid (0,25 Mol) in 285 g N-n-Propylpyrrolidon wurden bei 20°C 120 g Triethylamin-tris-hydrofluorid (0,75 Mol) getropft. Durch Anlegen von Oelvakuum und Erwärmen bis 70°C konnten in einer Kühlfalle 25 g (74% d. Th.) Cyanurfluorid erhalten werden.

**Patentansprüche**

1. Verfahren zur Herstellung organischer Fluorverbindungen durch Umsetzung de entsprechenden Halogenverbindungen, welche in wenigstens einer Position durch von Fluor verschiedenes Halogen substituiert sind, mit Fluoriden von Stickstoffbasen, dadurch gekennzeichnet, daß als Fluoride von

Stickstoffbasen die Hydrofluoride organischer Stickstoffbasen verwendent werden.

2. Verhahren nach Anspruch 1, dadurch gekennzeichnet, daß man als Ausgangshalogenverbindungen, welche an wenigstens einer Position durch von Fluor verschiedenes, nukleophil austauschlares Halogen substituiert sind, organische Chlor- und/oder Bromverbindungen verwendet.

3. Verfahren nach den Ansprüchen 1 und 2, dadurch gekennzeichnet, daß man als Ausgangshalogenverbindungen, welche an wenigstens einer Position durch von Fluor verschiedenes Halogen substituiert sind, Chlor- und/oder Bromalkane, $\alpha$-Chlor- und/oder -brom-ether, $\alpha$-Chlor- und/oder -brom-ketone, $\alpha$-Chlor und/oder -brom-carbon-säureester, Carbonsäurechloride und/oder -bromide sowie Chlor- und/oder Brom-aromaten und/oder -heterocyclen verwendet.

4. Verfahren nach Ansprüchen 1 bis 3, dadurch gekennzeichnet, daß man als Hydrofluoride organischer Stickstoffbasen die Hydrofluoride primärer, sekundärer und/oder teriärer, vorzugsweise sekundärer und/oder tertiärer, aliphatischer. Amine mit insgesamt bis zu 12 C-Atomen verwendet.

5. Verfahren nach den Ansprüchen 1 bis 4, dadurch gekennzeichnet, daß man die Umsetzung im Falle der Verwendung von solchen Hydrofluoriden organischer Stickstoffbasen, welche mer als ein Molekül Fluorwasserstoff pro Molekül organischer Stickstoffbase aufweisen, in Gegenwart eines basischen Lösungsmittels, vorzugsweise eines N—($C_1$—$C_4$)-alkylsubstituierten $C_5$- und/oder $C_6$-Lactams, durchführt.

6. Verfahren nach den Ansprüchen 1 bis 5, dadurch gekennzeichnet, daß man die Umsetzung bei Temperaturen zwischen etwa —10 und +150°C durchführt.

## Claims

1. A process for the manufacture of organic fluorine compounds by reaction of the corresponding halogen compounds substituted in at least one position by halogen other than fluorine, with fluorides of nitrogen bases characterized by using as fluorides of nitrogen bases hydrofluorides of organic nitrogen bases.

2. The process as claimed in claim 1, characterized by using as starting halogen comounds substituted in at least one position by nucleophilic exchangeable halogen other than fluorine organic chlorine and/or bromine compounds.

3. The process as claimed in claims 1 and 2, characterized by using as starting halogen compounds substituted in at least one position by halogen other than fluorine chloro- and/or bromoalkanes, alpha-chlor- and/or -bromoethers, alpha-chloro- and/or bromoketones, alpha-chloro- and/or bromocarboxylic acid esters, carboxylic acid chlorides and/or -bromides, and chloro and/or bromoaromatic and/or -heterocylic compounds.

4. The process as claimed in claims 1 to 3, characterized by using as hydrofluorides or organic nitrogen bases the hydrofluorides of primary, secondary and/or tertiary, preferably secondary and/or tertiary, aliphatic amines having a total of up to 12 carbon atoms.

5. The process as claimed in claims 1 to 4, characterized by carrying out the reaction in the case of using hydrofluorides of organic nitrogen bases containing more than one molecule of hydrogen fluoride per molecule of organic nitrogen base in the presence of a basic solvent, preferably a N—($C_1$—$C_4$)-alkylsubstituted $C_5$- and/or $C_6$-lactam.

6. The process as claimed in claims 1 to 5, characterized by carrying out the reaction at a temperature of from about —10 to +150°C.

## Revendications

1. Procédé de préparation de composés organiques fluorés par réaction des composés halogénés correspondants qui portent, en au moins une position, un halogène autre que le fluor, avec des fluorures de bases azotées, procédé caractérisé en ce qu'on utilise, comme fluorures de bases azotées, les fluorhydrates de bases azotées organiques.

2. Procédé selon la revendication 1, caractérisé en ce qu'on utilise des composés organiques chlorés et/ou bromés comme corps de départ portant, en au moins une position, un halogène autre que le fluor pouvant être échangé par une réaction nucléophile.

3. Procédé selon l'une des revendications 1 et 2, caractérisé en ce qu'on utilise, comme composés halogénés de départ portant, en au moins une position, un halogène autre que le fluor, des chloro- et/ou bromo-alcanes, des éthers $\alpha$-chlorés et/ou $\alpha$-bromés, des cétones $\alpha$-chlorées et/ou $\alpha$-bromées, des esters d'acides $\alpha$-chloro- et/ou $\alpha$-bromo-carboxyliques, des chlorures et/ou des bromures d'acides carboxyliques ainsi que des composés aromatiques et/ou hétérocycliques chlorés et/ou bromés.

4. Procédé selon l'une quelconque des revendications 1 à 3, caractérisé en ce qu'on utilise, comme fluorhydrates de bases azotées organiques, les fluorhydrates d'amines aliphatiques primaires, secondaires et/ou teriaires, de préférence secondiares et/ou tertiaires, contenant au plus 13 atomes de carbone en tout.

5. Procédé selon l'une quelconque des revendications 1 à 4, caractérisé en ce qu'on effectue la réaction, dan le cas où l'on utilise des fluorhydrates de bases azotées organiques contenant plus d'une

molécule de fluorure d'hydrogène par molécule de base azotée organique, en présence d'un solvant basique, de préférence d'un lactame en $C_5$ et/ou en $C_6$ portant un alkyle en $C_1$-$C_4$ à l'azote.

6. Procédé selon l'une quelconque des revendications 1 à 5, caractérisé en ce qu'on effectue la réaction à des températures comprises entre environ −10 et +150°C.